(19) Europäisches Patentamt
European Patent Office
Office européen des brevets

(11) EP 3 488 903 A1

(12) EUROPÄISCHE PATENTANMELDUNG

(43) Veröffentlichungstag:
29.05.2019 Patentblatt 2019/22

(51) Int Cl.:
A63B 71/06 (2006.01)          A63B 71/12 (2006.01)
A63B 71/14 (2006.01)          A63B 69/00 (2006.01)

(21) Anmeldenummer: 17203605.5

(22) Anmeldetag: 24.11.2017

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
MA MD

(71) Anmelder: Punch-IT GmbH
50931 Köln (DE)

(72) Erfinder:
• Goldammer, Wolfgang
  50931 Köln (DE)
• Cicek, Izzet
  50999 Köln (DE)

(74) Vertreter: dompatent von Kreisler Selting Werner -
Partnerschaft von Patent- und Rechtsanwälten
mbB
Deichmannhaus am Dom
Bahnhofsvorplatz 1
50667 Köln (DE)

(54) **VERFAHREN ZUR ERMITTLUNG DER KRAFTEINWIRKUNG EINER PERSON AUF EIN TRAININGSGERÄT**

(57) Bei einem Verfahren zur Ermittlung der Krafteinwirkung einer Person, insbesondere einer eine Kampfsportart ausführenden Person, auf ein rückstellfähiges Trainingsgerät, z.B. durch Einwirkung auf das Gerät mittels einer Extremität, wie z.B. der Hand oder des Fußes der Person, ist vorgesehen, dass
- ein rückstellfähiges Trainingsgerät bereitgestellt wird, bei dem die aus dem Ausmaß einer Auslenkung des Trainingsgeräts resultierende aufgebrachte Kraft und/oder eingebrachte Energie bekannt ist und/oder aus dessen physikalischen Eigenschaften ermittelbar ist,
- die in Richtung des Trainingsgeräts erfolgende Bewegung der Extremität der Person ab spätestens dem Zeitpunkt des Auftreffens der Extremität auf das Trainingsgerät bis zum Zeitpunkt des Stillstands der in der besagten Richtung erfolgten Bewegung messtechnisch erfasst wird,
- aus der zwischen den beiden zuvor genannten Zeitpunkten erfolgten Bewegung der Extremität der Person die Auslenkung des Trainingsgeräts und aus der Auslenkung des Trainingsgeräts die auf das Trainingsgerät ausgeübte Kraft und/oder die in das Trainingsgerät eingebrachte Energie ermittelt wird.

G

**Fig. 2**

EP 3 488 903 A1

**Beschreibung**

**1. Technisches Gebiet der Erfindung**

Die Erfindung betrifft ein Verfahren zur Ermittlung der Krafteinwirkung einer Person, insbesondere einer eine Kampfsportart ausführenden Person, auf ein rückstellfähiges Trainingsgerät, z.B. durch Einwirkung auf das Gerät mittels einer Extremität, wie z.B. der Hand/Faust oder des Fußes der Person.

Insbesondere betrifft die Erfindung die Ermittlung der Krafteinwirkung und anderer Schlagparameter bei Kampfsportarten über Bewegungssensoren in einem vom Sportler am Körper getragenen Gerät.

**2. Hintergrund**

Beim Training für Kampfsportarten kommt der Objektivierung von Trainingsfortschritten - wie in den meisten Sportarten - eine erhebliche Bedeutung zu. Wesentliche Trainingselemente sind Schläge oder Tritte auf hierzu bestimmte Trainingsgeräte (Sandsäcke, Makiwaras, ...). Hier ist es für den Sportler wünschenswert, die Stärke der Schläge (oder Tritte) sowie andere Parameter (z.B. Häufigkeit von Schlägen oder Tritten pro Zeitintervall) objektiv messen zu können, um sich mit anderen Sportlern zu vergleichen und um Trainingsfortschritte beurteilen und aufzeichnen zu können.

Bislang gibt es hierzu die Möglichkeit, mit Drucksensoren ausgestattete Trainingspads zu nutzen, über die eine Ermittlung und Anzeige der Schlagkraft erfolgen kann. Dies hat eine Reihe von Nachteilen:

• Die Messung der Schlagkraft ist nur für bestimmte Trainingseinheiten möglich (Schläge oder Tritte auf ein feststehendes Pad). Bei Nutzung vieler Trainingsgeräte (z.B. Sandsäcke, Makiwaras) kann die Schlagkraftermittlung auf diese Weise nicht erfolgen.

• Die Ermittlung der Schlagparameter ist auf die Schlagkraft beschränkt. Weitere Parameter wie die Schlaggeschwindigkeit lassen sich nicht oder (wie z.B. die Häufigkeit von Schlägen) nur mit Aufwand ermitteln.

• Die Ermittlung der Schlagparameter erfolgt nicht personalisiert für den Sportler, sondern wird lediglich in der Trainingseinrichtung angezeigt.

Darüber hinaus gibt es Konzepte zur Ermittlung von Bewegungsabläufen und Schlagparametern über mit Beschleunigungssensoren ausgestattete Geräte, die vom Sportler am Körper getragen werden (z.B. Boxhandschuhe mit integrierten Sensoren). Dies erlaubt die Bestimmung der Schlagfrequenz und aus den Beschleunigungsdaten kann die Geschwindigkeit des Schlages ermittelt werden.

Ohne Bezug zu den Eigenschaften und Materialparametern der verwendeten Trainingsgeräte lässt sich aus diesen Daten aber nicht die tatsächlich auf das Trainingsgerät ausgeübte Kraft ermitteln, da eine unmittelbare Verwendung der Gesetzmäßigkeit Kraft = Masse x Beschleunigung nicht zur Ermittlung der ausgeübten Kraft verwendet werden kann:

• Die beschleunigte Masse ist nicht genau definierbar (neben der Schlaghand wird auch ein nicht genau definierbarer Teil des Körpers beschleunigt und beim Schlag abgebremst).

• Eine Anwendung dieser Formel würde dem gedachten Fall entsprechen, dass ein Gegenstand (z.B. ein Ball) mit der entsprechenden Geschwindigkeit und Masse gegen das Trainingsgerät geworfen wird und dann abgebremst wird. Eine Ermittlung der Kraft über diesen Zusammenhang vernachlässigt daher, dass während des Schlages eine weitere beschleunigende Muskelkraft wirkt, die der Verzögerung durch das Trainingsgerät entgegen wirkt. Diese zusätzliche Muskelkraft wird in der obigen Formel nicht berücksichtigt.

Aufgabe der Erfindung ist es, ein Verfahren zur Ermittlung der Krafteinwirkung einer Person auf ein rückstellfähiges Trainingsgerät, mit dem sich die Kraft, die auf das Trainingsgerät wirkt, und/oder die Energie, die in das Trainingsgerät eingebracht worden ist, genauer ermitteln lässt bzw. lassen.

Zur Lösung dieser Aufgabe wird mit der Erfindung ein Verfahren vorgeschlagen, wobei

- ein rückstellfähiges Trainingsgerät bereitgestellt wird, bei dem die aus dem Ausmaß einer Auslenkung des Trainingsgeräts aus einer Ruheposition resultierende aufgebrachte Kraft und/oder eingebrachte Energie bekannt ist und/oder aus dessen physikalischen Eigenschaften ermittelbar ist,

- die in Richtung des Trainingsgeräts erfolgende Bewegung der Extremität der Person ab spätestens dem Zeitpunkt des Auftreffens der Extremität auf das Trainingsgerät bis zum Zeitpunkt des Stillstands der in der besagten Richtung erfolgten Bewegung messtechnisch erfasst wird,

- aus der zwischen den beiden zuvor genannten Zeitpunkten erfolgten Bewegung der Extremität der Person die Auslenkung des Trainingsgeräts und aus der Auslenkung des Trainingsgeräts die auf das Trainingsgerät ausgeübte Kraft und/oder die in das Trainingsgerät eingebrachte Energie ermittelt wird.

In vorteilhafter Weiterbildung der Erfindung kann vorgesehen sein, dass die in Richtung auf das Trainingsgerät erfolgende Bewegung der Extremität der Person ab dem Zeitpunkt des Beginns der Bewegung messtechnisch erfasst wird.

Besonders vorteilhaft kann es sein, wenn die Bewegung der Extremität der Person anhand von mindestens einem zumindest eindimensionalen, vorzugsweise dreidimensionalen Sensor, insbesondere Weg- oder Beschleunigungssensor, Gyrosensor und/oder Magnetometer, messtechnisch erfasst wird.

Zweckmäßig kann es ferner sein, wenn der mindestens eine Sensor von der Person an deren auf das Trainingsgerät einwirkenden Extremität getragen wird.

Die Nachteile des Standes der Technik werden durch das erfindungsgemäße Verfahren überwunden, bei dem die Schlagparameter nicht direkt, sondern indirekt über entsprechende Sensoren ermittelt werden. Die interessierenden Parameter (insbesondere die auf den Schlaggegenstand ausgeübte Kraft) werden dabei aus den Sensordaten über die Nutzung physikalischer Zusammenhäng bestimmt. Diese Zusammenhänge erlauben neben der Bestimmung der während des Schlages oder Tritts wirkenden Kraft auch die Berechnung der vom Sportler aufgewendeten Energie.

Das erfindungsgemäße Verfahren ist bei einer breiten Palette von Trainingsgeräten anwendbar und erfordert für jedes Trainingsgerät lediglich die Festlegung von wenigen Kalibrierparametern (z.B. Masse und Länge der Aufhängung des Trainingsgeräts, auf das durch Schlagen eingewirkt wird, wie z.B. bei Sandsäcken, oder Federkonstante, wie z.B. bei Makiwaras).

Die Daten werden über ein mit Bewegungssensoren ausgestattetes Gerät (sogenanntes Wearable) ermittelt und analysiert, das vom Sportler je nachdem, ob die Kraft im Arm oder im Bein gemessen werden soll, am Arm- oder Fußgelenk getragen wird. Über eine entsprechende drahtlose Kommunikation können die Daten an ein Empfangsgerät übermittelt werden, wo sie weiteren Auswertungen unterzogen, dem Sportler dargestellt, gespeichert und optional an eine Cloud gesendet werden.

## 3. Anwendungsbereich

Das entwickelte Konzept ist auf alle Kampfsportarten anwendbar, bei denen ein Sportler gegen einen Gegenstand schlägt oder tritt, wobei der Gegenstand dem Schlag oder Tritt über seine elastischen Eigenschaften oder über die Schwerkraft einen Widerstand entgegensetzt. In diesen Fällen können die auf den Gegenstand ausgeübte Kraft, die übertragene Energie sowie weitere Trainingsparameter wie die Frequenz und die Gesamtzahl von Schlägen oder Tritten bestimmt werden.

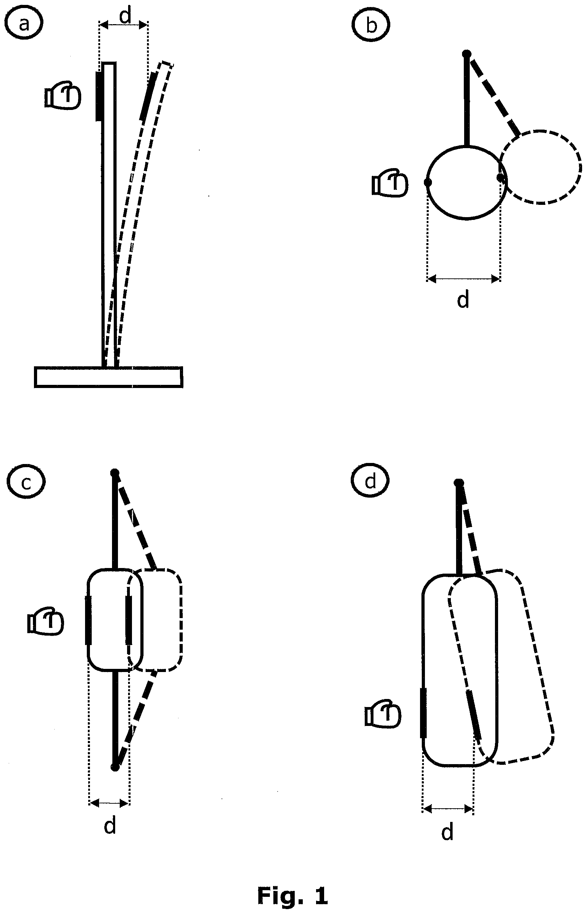

In Fig. 1 sind häufig verwendete Trainingsgeräte dargestellt, bei denen die Erfindung anwendbar ist.

Bei Makiwaras (Fig. 1a) oder ähnlichen Geräten erfolgt der Schlag gegen einen elastischen Gegenstand (z.B. ein Brett). Dieser Gegenstand wird durch den Schlag über eine bestimmte Strecke d ausgelenkt. Die hierzu erforderliche Kraft ergibt sich aus der Auslenkung d und den Materialeigenschaften, die sich in der Federkonstante K widerspiegeln. Über die Gesetzmäßigkeit Energie = Kraft x Weg lässt sich zudem die übertragene Energie bestimmen.

An elastischen Halterungen aufgehängte Gegenstände (Fign. 1b und 1c) entsprechen in ihren physikalischen Eigenschaften Makiwaras und lassen sich analog behandeln.

Bei einem Sandsack (Fig. 1d) wird dieser durch den Schlag beschleunigt und ebenfalls um eine Strecke d ausgelenkt. Aus dieser Auslenkung d lässt sich die übertragene Energie bestimmen, wenn Masse M und Länge der Aufhängung L bekannt sind. Diese Energie erlaubt den Rückschluss auf die beim Schlag oder Tritt wirkende Kraft sowie die übertragene Energie.

## 4. Funktionsprinzip

Die Ermittlung der Schlagparameter erfolgt auf indirektem Weg über eine Analyse der Schlagbewegung (oder der Trittbewegung). Hierzu gehört zum einen die Beschleunigungsphase von Hand oder Fuß. Beim Auftreffen auf das Trainingsgerät entsteht dann eine starke Verzögerung der Bewegung. Die raum- und zeitaufgelöste Ermittlung der Position und Geschwindigkeit von Hand oder Fuß während der Schlagbewegung bildet dann zusammen mit Materialparametern des Schlaggegenstands die Basis der Auswertungen, deren Methodik im Folgenden spezifiziert wird.

## 4.1 Ermittlung der zeitaufgelösten Koordinaten der Schlagbewegung

Die zunächst zu ermittelnden Zielparameter während Beschleunigung und Abbremsung von Hand oder Fuß sind die zeitaufgelösten Koordinaten der Schlagbewegung auf den drei Raumachsen relativ zum Trainingsgerät.

Als Basis für deren Bestimmung werden die von dem Beschleunigungssensor in kurzen Zeitintervallen in der Größenordnung von z.B. Millisekunden gelieferten Messwerte der Beschleunigung verwendet. Über eine Rückrechnung (Integration) auf die Geschwindigkeit und weiter auf die von Hand oder Fuß zurückgelegte Entfernung lässt sich hieraus die Schlagbewegung über zeitaufgelöste dreidimensionale Koordinatenwerte beschreiben:

- Für jedes Zeitintervall ergibt sich dabei der Vektor der zurückgelegten Entfernung abhängig von der räumlichen Anfangsposition und der Geschwindigkeit von Hand oder Fuß zu Beginn des Zeitintervalls.
- Die Veränderung der Geschwindigkeit im Zeitintervall wird aus den Beschleunigungswerten berechnet. Die so ermittelten Entfernungs- und Geschwindigkeitsvektoren bilden dann die Basis für die Auswertung der Schlagbewegung im nächsten Zeitintervall.
- Die vor Beginn des Schlages vorliegenden Anfangswerte zur räumlichen Position und Geschwindigkeit lassen sich aus den Beschleunigungsdaten nicht berechnen. Aus der Rückrechnung der Entfernungs- und Geschwindigkeitsvektoren während der gesamten Schlagbewegung lassen sich diese Anfangswerte aber relativ zum Trainingsgerät bestimmen und dies ergibt eine ausreichende Basis für die weiteren Auswertungen der Schlagparameter. Ausgangspunkt hierfür sind die ermittelten Daten zum Auftreff- und Umkehrpunkt (s. Abschnitt 4.2).

Da ein Schlag oder Tritt in der Praxis nicht völlig linear in Richtung des Trainingsgeräts erfolgt, kann eine Steigerung der Genauigkeit der Ermittlung der zeitlich aufgelösten Entfernungs- und Geschwindigkeitsvektoren dadurch erfolgen, dass neben den Beschleunigungswerten über einen Beschleunigungssensor auch die Drehbewegung über einen Gyrosensor bestimmt wird. Außerdem besteht zur weiteren Steigerung der Genauigkeit die Option, anstelle oder zusätzlich zu dem Gyrosensor ein Magnetometer einzusetzen. Eine weitere Genauigkeitssteigerung kann dadurch erreicht werden, dass zwei Bewegungssensoren verwendet werden, die jeweils für niedrige bzw. hohe Beschleunigungen ausgelegt sind und in den verschiedenen während des Schlages auftretenden Beschleunigungsbereichen genauere Daten liefern.

Insgesamt können die ggf. durch die mehreren Sensoren ermittelten Messwerte verwendet werden, um die Bestimmung der zeitaufgelösten dreidimensionalen Koordinaten von Hand und Fuß sowie der jeweiligen Geschwindigkeit während des Schlages genauer zu bestimmen.

**4.2 Ermittlung der primären Schlag- oder Trittparameter**

Um die Schlagparameter zu berechnen, müssen zunächst die folgenden Zeitpunkte ermittelt werden:

• Beginn der Schlagbewegung;
• Auftreffzeitpunkt von Hand oder Fuß auf das Trainingsgerät und
• Umkehrzeitpunkt, an dem die maximale Auslenkung d (siehe Fig. 1) erreicht ist.

Diese Zeitpunkte lassen sich aus der zeitlich aufgelösten Analyse der Schlag- oder Trittbewegung gemäß Abschnitt 4.1 bestimmen:

• Der Beginn der Schlagbewegung lässt sich aus dem Anfang der Beschleunigungsphase von Hand oder Fuß ermitteln.
• Der Zeitpunkt des Auftreffens von Hand oder Fuß auf das Trainingsgerät wird als Beginn der Verzögerung bestimmt.
• Zum Ende des Schlages am Umkehrzeitpunkt erfolgt eine Umkehr der Bewegungsrichtung, die sich ebenfalls aus den zeitaufgelösten dreidimensionalen Koordinaten von Hand und Fuß ablesen lässt.

Die ermittelten Zeitpunkte bilden zum einen die Basis der Bestimmung der Schlagfrequenz. Zum anderen ermöglicht die Ermittlung der Auslenkung d des Trainingsgeräts zwischen Auftreff- und Umkehrpunkt die Ermittlung von ausgeübter Kraft und übertragener Energie (s. Abschnitt 4.3).

Die konkrete Durchführung dieser Analyse zur Ermittlung der primären Schlag- oder Trittparameter kann auf analytischem Wege erfolgen, indem die zeitaufgelösten Ergebnisse aus Abschnitt 4.2 ausgehend von gewissen Schwellenwerten der Beschleunigung bzw. Verzögerung zur Ermittlung der verschiedenen Schlagphasen verwendet werden.

Eine Alternative besteht darin, die zeitlichen Parameter des Schlages oder Trittes mit Methoden der Künstlichen Intelligenz zu ermitteln. Die gemäß Abschnitt 4.1 ermittelten Daten zur zeitaufgelösten Beschleunigung können als Eingangswerte eines neuronalen Netzes verwendet werden. Zusätzlich oder alternativ können auch hieraus abgeleitete Werte (Geschwindigkeit und zurückgelegte Entfernung) an ein neuronales Netz übergeben werden.

Nach einem entsprechenden Training des neuronalen Netzes mit Daten zu realen Schlägen kann dieses verwendet werden, um alle oder einige der o.g. Schlagparameter zu ermitteln. Dies gilt sowohl für isolierter Schläge oder Tritte wie auch für Serien von Schlägen oder Tritten, aus denen ein entsprechend trainiertes neuronales Netz die Einzelereignisse herausfiltern und deren primäre Parameter in Form der Zeitpunkte der Einzelereignisse ableiten kann.

**4.3 Ermittlung der ausgeübten Kraft und übertragenen Energie**

Aus den primären Parametern der Schlag- oder Trittbewegung lässt sich über physikalische Zusammenhänge die auf das Trainingsgerät ausgeübte Kraft wie auch die übertragene Energie (d.h. geleistete Arbeit) bestimmen, die sich jeweils aus der Art des Trainingsgegenstandes ergeben:

• Bei Makiwaras oder ähnlich elastisch konzipierten oder aufgehängten Trainingsgeräten (s. Fign. 1a bis 1c) ergibt sich die aufzuwendende Kraft für die Auslenkung d als Produkt aus dieser Auslenkung und der Federkonstante K:

$$F = K * d$$

Die Federkonstante stellt in diesem Fall einen zu konfigurierenden Materialparameter dar, die Auslenkung d wird gemäß den vorherigen Abschnitten bestimmt. Die aufzuwendende Energie bestimmt sich in diesem Fall aus der Gleichung

$$E = \tfrac{1}{2} * K * d^2$$

- Bei einem Sandsack entspricht die aufzuwendende Kraft der Tangentialkomponente des Gewichtsvektors und die geleistete Arbeit der Erhöhung der potentiellen Energie. Beides lässt sich aus dem Auslenkungswinkel f des Sandsacks bezogen auf die Vertikale ermitteln. Dieser Auslenkungswinkel f ergibt sich aus der Auslenkung des Schwerpunkts $d_S$ und der Länge der Aufhängung von der Deckenbefestigung bis zum Schwerpunkt $L_S$ als

$$f = 2 * \arcsin (d_S / 2*L_S)$$

Da der Auftreffpunkt des Schlags oder Tritts nicht notwendig auf Schwerpunkthöhe liegt, muss hierbei die Auslenkung des Schwerpunkts $d_S$ aus der gemäß den vorherigen Abschnitten bestimmten Auslenkung d durch Multiplikation mit dem Verhältnis des Abstandes von Schwerpunkt $L_S$ und Auftreffpunkt $L_A$ von der Aufhängung bestimmt werden:

$$d_S = d * (L_S / L_A)$$

Aus dem so bestimmten Auslenkungswinkel f ergibt sich dann mit der Masse des Sandsacks M und der Erdbeschleunigung g die aufzuwendende Tangentialkomponente der Gewichtskraft als:

$$F = M * g * \sin(f)$$

Die aufzuwendende Energie ergibt sich aus der potenziellen Energie des Schwerpunkts bei der Auslenkung um den Winkel f zu:

$$E = M * g * L_S * (1 - \cos(f))$$

Damit erfordert die Umsetzung bei Makiwaras oder ähnlichen elastisch reagierenden Trainingsgeräten die Bestimmung der Federkonstante, die z.B. aus einer einfachen Messung der Auslenkung mit einer Zugwaage zur Kraftbestimmung ermittelt werden kann. Für Sandsäcke sind neben der im Regelfall bekannten Masse die leicht zu messenden Abstände von Schwerpunkt und Auftreffpunkt von der Aufhängung zu ermitteln.

**4.4 Beispiele für die technische Realisierung**

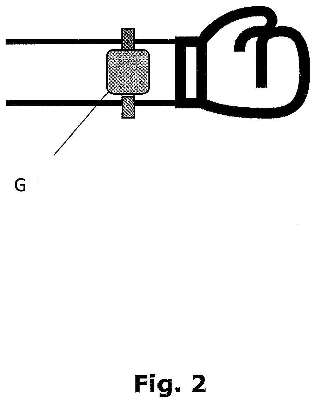

Die zu verwendenden Sensoren (Beschleunigungssensoren und ggf. weitere unterstützende Sensoren wie Gyrosensor und Magnetometer) stehen als Standardkomponenten in Form von integrierten Schaltkreisen (MEMS) zur Verfügung. Sie sind in einem am Schlagarm (oder Unterarm oder Handgelenk) oder Schlagfuß (Fußgelenk) oder zugehörigen Bein (Ober- oder Unterschenkel) zu tragenden Gerät G angeordnet (s. Fig. 2). Bei dem Gerät handelt es sich um ein Wearable, wie z.B. Fitness- oder Activity-Tracker mit der für die Erfindung erforderlichen Bewegungs- und/oder Beschleunigungssensorik (vorzugsweise 3D-Sensorik) oder um ein Smartphone mit einer solchen Sensorik.

Zur Auswertung der Sensordaten sollten diese mit genauer Zeitauflösung im Millisekunden-Bereich ermittelt und analysiert werden. Hierzu ist in dem Gerät ein entsprechender Zeitmesser erforderlich.

Weiter müssen die analogen Daten digital transformiert werden und dann in einer CPU ausgewertet werden. Dies erfolgt entweder über analytische Algorithmen oder ganz oder teilweise durch Nutzung eines neuronalen Netzes, das über entsprechende Softwaretools in der CPU implementiert wird.

Außerdem muss ein lokaler Speicher für die Programmkomponenten und die notwendigen Parameter vorhanden sein. Hierzu gehören die Basisparameter der verwendeten Trainingsgeräte. Bei Nutzung eines neuronalen Netzes schließen diese Parameter auch die über das Lernen ermittelten Verbindungsgewichte der Neuronen ein.

Hinzu kommen weitere Komponenten zur Stromversorgung und Anzeige- und Bedieneinrichtungen.

Der Start der Auswertungsphasen erfolgt entweder über Tasten bzw. berührungsempfindliche Sensoren in dem am Körper getragenen Gerät oder über Gestensteuerung (z.B. Hin- und Herdrehen des Handgelenks zum Start einer Schlagauswertung).

Die Daten werden in dem Gerät per Software aufbereitet. Bei Nutzung einer Anzeigeeinrichtung am Gerät werden ermittelte Kerndaten der Analyse dem Benutzer unmittelbar angezeigt. Zusätzlich besteht die Option, die Daten zur weiteren Auswertung und Verwendung über eine drahtlose Kommunikationsverbindung (z.B. Bluetooth) an ein Basisgerät zu übertragen. Über die Verbindung mit einem Basisgerät kann auch die Konfiguration der Basisparameter zu den Trainingsgeräten erfolgen.

Auf dem Basisgerät können die Daten dann über die Nutzung entsprechender Apps weiter aufbereitet, gespeichert und mit Daten früherer Trainingseinheiten oder anderer Sportler verglichen werden. Es besteht auch die Möglichkeit, Speicherung und Vergleich von Analysedaten über das Internet zu organisieren.

**4.5 Zusammenfassung**

Bei dem erfindungsgemäßen Verfahren kann aus den Beschleunigungswerten und ggf. weiteren Sensorinformationen durch Ermittlung der physikalischen Kenngrößen des Schlags oder Tritts die erzielte Auslenkung des Trainingsgeräts ermittelt werden. Aus den physikalischen Eigenschaften des Trainingsgeräts kann mit Hilfe von Kalibrierparametern (z.B. Masse und Länge der Aufhängung bei Sandsäcken oder Federkonstante bei Makiwaras) durch Anwendung der entsprechenden physikalischen Gesetzmäßigkeiten die Schlagstärke in Newton bestimmt werden. Weitere Parameter wie die Schlagfrequenz sowie Bewegungsparameter z.B. der Ausholbewegung lassen sich ebenfalls durch Rückrechnung aus den Beschleunigungswerten ermitteln. Ebenfalls bestimmen lässt sich aus diesen Daten die an den Trainingsgegenstand übertragene Energie.

**Patentansprüche**

1. Verfahren zur Ermittlung der Krafteinwirkung einer Person, insbesondere einer eine Kampfsportart ausführenden Person, auf ein rückstellfähiges Trainingsgerät, z.B. durch Einwirkung auf das Gerät mittels einer Extremität, wie z.B. der Hand oder des Fußes der Person, wobei bei dem Verfahren

   - ein rückstellfähiges Trainingsgerät bereitgestellt wird, bei dem die aus dem Ausmaß einer Auslenkung des Trainingsgeräts aus einer Ruheposition resultierende aufgebrachte Kraft und/oder eingebrachte Energie bekannt ist und/oder aus dessen physikalischen Eigenschaften ermittelbar ist,
   - die in Richtung des Trainingsgeräts erfolgende Bewegung der Extremität der Person ab spätestens dem Zeitpunkt des Auftreffens der Extremität auf das Trainingsgerät bis zum Zeitpunkt des Stillstands der in der besagten Richtung erfolgten Bewegung messtechnisch erfasst wird,
   - aus der zwischen den beiden zuvor genannten Zeitpunkten erfolgten Bewegung der Extremität der Person die Auslenkung des Trainingsgeräts und aus der Auslenkung des Trainingsgeräts die auf das Trainingsgerät ausgeübte Kraft und/oder die in das Trainingsgerät eingebrachte Energie ermittelt wird.

2. Verfahren nach Anspruch 1, **dadurch gekennzeichnet, dass** die in Richtung auf das Trainingsgerät erfolgende Bewegung der Extremität der Person ab dem Zeitpunkt des Beginns der Bewegung messtechnisch erfasst wird.

3. Verfahren nach Anspruch 1 oder 2, **dadurch gekennzeichnet, dass** die Bewegung der Extremität der Person anhand von mindestens einem zumindest eindimensionalen, vorzugsweise dreidimensionalen Sensor, insbesondere Weg- oder Beschleunigungssensor, Gyrosensor und/oder Magnetometer, messtechnisch erfasst wird.

4. Verfahren nach Anspruch 3, **dadurch gekennzeichnet, dass** der mindestens eine Sensor von der Person, an deren auf das Trainingsgerät einwirkenden Extremität getragen wird.

**Fig. 1**

G

**Fig. 2**

Europäisches
Patentamt

European
Patent Office

Office européen
des brevets

## EUROPÄISCHER RECHERCHENBERICHT

**Nummer der Anmeldung**

EP 17 20 3605

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2014/248594 A1 (NAVAS VICTOR XAVIER [US]) 4. September 2014 (2014-09-04) * Absatz [0015] - Absatz [0016] * * Absatz [0015] - Absatz [0018] * ----- | 1-4 | INV. A63B71/06 A63B71/12 A63B71/14 A63B69/00 |
| A | US 2016/263458 A1 (MATHER GEOFFREY [US] ET AL) 15. September 2016 (2016-09-15) * Abbildungen * * Absatz [0158] - Absatz [0159] * * Absatz [0098] - Absatz [0100] * ----- | 1-4 | |
| A | DE 10 2011 085719 A1 (BOSBACH WALTER [DE]) 8. Mai 2013 (2013-05-08) * Abbildungen * ----- | 1 | |
| A | US 2014/372440 A1 (CAINS STEVEN [GB]) 18. Dezember 2014 (2014-12-18) * Absatz [0054] - Absatz [0059]; Abbildungen * ----- | 1 | |

**RECHERCHIERTE SACHGEBIETE (IPC)**

A63B

Der vorliegende Recherchenbericht wurde für alle Patentansprüche erstellt

| Recherchenort | Abschlußdatum der Recherche | Prüfer |
|---|---|---|
| München | 14. Juni 2018 | Lundblad, Hampus |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

    ..................................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 20 3605

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-06-2018

| Im Recherchenbericht angeführtes Patentdokument | Datum der Veröffentlichung | Mitglied(er) der Patentfamilie | Datum der Veröffentlichung |
|---|---|---|---|
| US 2014248594 A1 | 04-09-2014 | KEINE | |
| US 2016263458 A1 | 15-09-2016 | KEINE | |
| DE 102011085719 A1 | 08-05-2013 | KEINE | |
| US 2014372440 A1 | 18-12-2014 | KEINE | |

Für nähere Einzelheiten zu diesem Anhang : siehe Amtsblatt des Europäischen Patentamts, Nr.12/82

EPO FORM P0461